# EUROPEAN PATENT APPLICATION

(11) **EP 1 712 292 A1**
(43) Date of publication of application: **18.10.2006**
(21) Application number: 05102846.2
(22) Date of filing: 11.04.2005
(51) Int. Cl.: B05B 11/00, A61F 9/00

(54) **Dispensing device for delivering fluid having a dosing chamber and a slide valve assembly**

(71) Applicant: Helbling Technik Bern AG, 3097 Liebefeld-Berne (CH)
(72) Inventor: Péclat, Christian, 2000 Neuchâtel (CH); Fontannaz, Joel, 1926 Fully (CH)
(74) Representative: Robert, Vincent

(57) **Abstract**

The present invention provides a dispensing device (10) for applying fluid comprising a pressurized reservoir equipped with a dosing module (20) having at least one dosing chamber which is able to communicate with the reservoir and with a nozzle (54) provided for dispensing a dose of fluid, characterized in that the dosing chamber comprises a moving and/or deformable member which delimits at least a first and a second compartments of variable volume, each compartment having an inlet and an outlet associated with valve means, wherein valve means are controlled according to a first configuration in which the inlet of the first compartment and the outlet of the second compartment are open while the other inlet and outlet being closed, and according to a second configuration in which the inlet of the second compartment and the outlet of the first compartment are open while the other inlet and outlet being closed.

## Description

### BACKGROUND OF THE INVENTION

The present invention relates generally to a device for dispensing a dose of fluid.

The present invention relates more particularly to a dispensing device for applying fluid comprising a pressurized reservoir equipped with a dosing module, the dosing module having at least one dosing chamber which is able to communicate, on one hand, with the reservoir and, on the other hand, with at least one nozzle provided for dispensing a dose of fluid.

This type of dispensing device is particularly useful for delivering ophthalmic drugs topically. Such a dispensing device is disclosed, for example, in document WO-A-98/12511, wherein a piston slidable between a first and a second priming position, upon action of a pre-loaded spring, is actuated by means of a trigger in order to cause a fluid charge to be expelled through a nozzle.

This type of dispensing device is not completely convenient because it comprises a lot of components, the precision of the dose delivering is not guaranteed, the complexity of the mechanism causes reliability problems and high fabrication costs.

The present invention is aimed to solve the above problems and others.

### SUMMARY OF THE INVENTION

According to the present invention there is provided a dispensing device for applying fluid comprising a pressurized reservoir equipped with a dosing module, the dosing module having at least one dosing chamber which is able to communicate, on one hand, with the reservoir and, on the other hand, with at least one nozzle provided for dispensing a dose of fluid, characterized in that the dosing chamber comprises a moving and/or deformable member which delimits at least a first and a second compartments of variable volume, each compartment having an inlet and an outlet associated with valve means, wherein valve means are controlled according to a first configuration in which the inlet of the first compartment and the outlet of the second compartment are open while the other inlet and outlet being closed, and according to a second configuration in which the inlet of the second compartment and the outlet of the first compartment are open while the other inlet and outlet being closed, such that, in the first configuration, the first compartment is refilled with a dose of fluid, under reservoir pressure, while the dose of fluid contained in the second compartment is expelled and, in the second configuration, the second compartment is refilled with a dose of fluid, under reservoir pressure, while the dose of fluid contained in the first compartment is expelled.

The dispensing device according to the present invention provides a great ability to deliver a precise dose of drug independently from the orientation of the device, contrarily to conventional eye droppers.

The dispensing device according to the present invention prevents a patient to influence or to compromise dose size by incorrect manipulations.

The characteristics of the product stream produced by each nozzle permit to overcome the blink reflex of the eyes.

The structure of the dispensing device according to the present invention permits extremely small dimensions for the device. For example, a dispensing device of three milliliters (typically one hundred doses) can be as small as about fifteen cubic centimeters.

The characteristics of the dispensing device make the device particularly compact and easy to use compared to usual eye droppers and also to other devices. It is very convenient for elderly people, children and persons with reduced mobility, avoiding assistance of a second person for drug administration.

The dispensing device according to the present invention is provided with a fully disposable fluidic path, which can be fabricated for very low cost. Hence, the dispensing device can be made only with plastic components and a metallic or plastic spring, without requiring any other additional energy source.

Thanks to its simple structure, the dispensing device can be easily safely secured for shelf life and shipment. During shipment, the drug fluid is separated from the dosing module by the septum which avoids fluid degradation and contamination.

### BRIEF DESCRIPTION OF THE DRAWINGS

The preferred embodiment of this invention will be described in detail, with reference to the following figures, wherein like designations denote like elements, and wherein:
Figure 1 shows schematically an axial section of a dispensing device in accordance with a preferred embodiment of the present invention;
Figure 2 shows schematically an exploded perspective view of the dispensing device of figure 1;
Figure 3 shows schematically an axial section of the dosing module equipping the dispensing device of figure 1;
Figure 4 is a view similar to figure 1 showing the dispensing device in a starting configuration before use;
Figure 5 is a view similar to figure 1 showing the dispensing device in operation with the slider in an intermediate position;
Figure 6 is a view similar to figure 1 showing the dispensing device in operation with the slider in its lower position just before the stream of fluid is released;
Figure 7 is a view similar to figure 1 showing the dispensing device releasing a dose of fluid;
Figure 8 is a view similar to figure 1 showing the dispensing device at the end of operation;
Figure 9 is an axial section of a perspective view showing the dispensing device provided with a casing.

### DETAILED DESCRIPTION

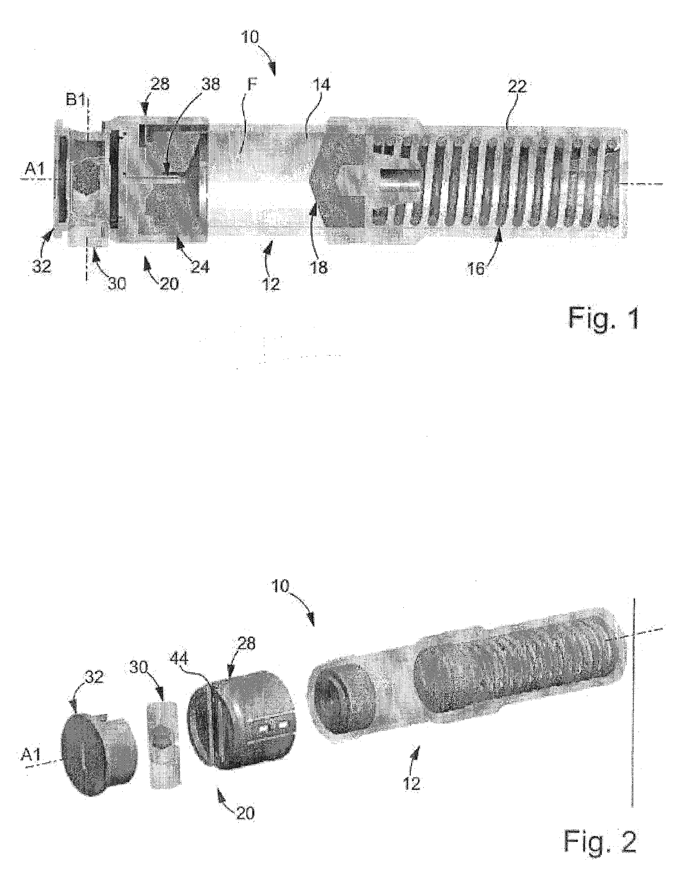

Referring to figure 1 to 2, a dispensing device 10 for delivery of ophthalmic drug in accordance with a preferred embodiment of the present invention is shown.

The dispensing device 10 is based on use of a pressurized reservoir 14 (drug primary packaging), preferably but not limited to a cylindrical glass cartridge 12 with a spring loaded piston 18, and a self-dosing delivery module 20 namely the dosing module 20.

The cartridge 12 comprises a tubular end portion 22 housing a pre-loaded spring 16 biasing the piston 18 axially towards the dispensing front end 24 of the cartridge 12. The dispensing front end 24 is initially sealed with a septum 26. The reservoir 14 contains the ophthalmic drug in the form of a fluid F to be expelled.

For comprehension purpose, an axial orientation from rear to front, along the cartridge axis A1, will be used.

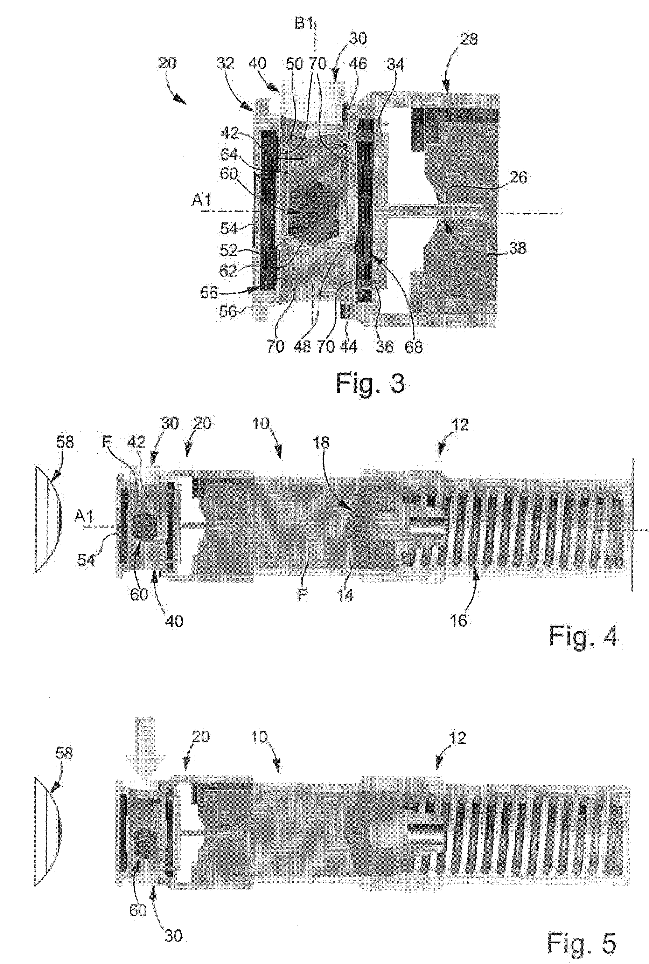

The dosing module 20, as shown on figure 3, comprises a support member 28 in the form of a cap which is clipped on the dispensing front end 24 of the cartridge 12, a slide valve assembly 30, and a front cap 32 which is mounted on the front end of the support member 28.

The support member 28 comprises an upper 34 and a lower 36 channels in communication with a tubular needle 38 which extends axially at a rear face of the support member 28 in order to pierce the septum 26 so as to set the dispersion device 10 operational.

The slide valve assembly 30 comprises a tubular slider 40 delimiting an inner dosing chamber 42 which is able to be in communication with the reservoir 14 through the channels 34, 36.

The slider 40 is slidably mounted in a guide groove 44 arranged on the front face of the support member 28, along a sliding axis B1 transversal to the cartridge axis A1.

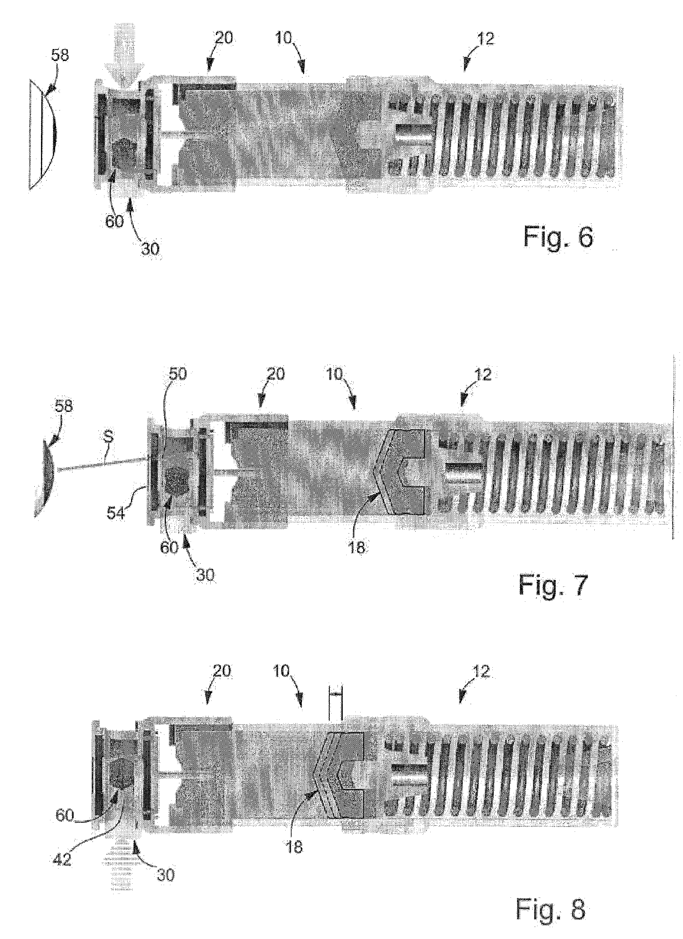

The slider 40 is able to move between two indexed positions, a lower and an upper positions, upon manual actuation such as finger pressure on one transverse end of the slider 40. Figure 3 shows the slider 40 in its upper position, and figure 8 shows the slider 40 in its lower position.

Preferentially, the slider 40 is bistable such that it can not stay in an intermediate position between its two indexed positions. A mechanical feature such as a hard point can be provided in the slide valve assembly 30 in order to prevent the slider 40 to stay in an intermediate position.

The slider 40 comprises an upper 46 and a lower 48 inlets which are able to match with the orifices of the upper 34 and the lower 36 channels of the support member 28 when the slider 40 is in its upper and in its lower positions respectively.

The slider 40 comprises an upper 50 and a lower 52 outlets which are able to communicate with the opening 54 arranged in the front face 56 of the front cap 32, when the slider 40 is in its lower and in its upper positions respectively.

In a different construction, the outlets 50 and 52 can be joined via a fluidic channel built in the slider 40, so that the dispensing device 10 has only one output nozzle instead of two.

According to the present embodiment, each outlet 50, 52 constitutes a nozzle designed for producing a stream S of fluid F through the opening 54 and towards an eye 58 of the patient. Each outlet 50, 52 has a general axis of projection which is inclined relatively to the cartridge axis A1 and which is directed towards the cartridge axis A1 such that, when the dispensing device 10 is well positioned in front of the eye 58, the stream S is directed towards the center of the eye 58. Inclination of the general axis of projection improves the precision of delivery.

The support member 28 and the front cap 32 are provided with some sealing surfaces 70 selectively facing external orifices of the inlets 46, 48 and of the outlets 50, 52 when the slider 40 occupies some given transversal positions, in order to close selectively the inlets 46, 48 and the outlets 50, 52.

According to a first configuration, wherein the slider 40 is in its upper position, the sealing surfaces 70 are closing the upper outlet 50 and the lower inlet 48 and, according to a second configuration, wherein the slider 40 is in its lower position, the sealing surfaces 70 are closing the lower outlet 50 and the upper inlet 46.

When the slider 40 is in an intermediate position, illustrated by figure 5, the sealing surfaces 70 are closing every inlet 46, 48 and every outlet 50, 52.

According to the present embodiment, the sealing surfaces 70 belong to a front 66 and to a rear 68 inserts arranged respectively on the rear face of the front cap 32 and on the front face of the support member 28.

The slider 40 comprises a shuttle plunger 60 which is slidably mounted in the dosing chamber 42, coaxially to the sliding axis B1 of the slider 40, between an upper position in which the shuttle plunger 60 closes the upper outlet 50 and the upper inlet 34, and a lower position in which the shuttle plunger 60 closes the lower outlet 52 and the lower inlet 36.

The shuttle plunger 60 delimits, in the dosing chamber 42, an upper compartment and a lower compartment of variable volume.

The volume of fluid F which can be contained in one compartment of the dosing chamber 42, when the shuttle plunger 60 occupies its upper or its lower position, matches with a given dose D of fluid F to be dispensed to the eye 58 of a patient.

The given dose D is defined solely by geometry, i.e. by the chamber dimension (diameter) and by the shuttle plunger stroke.

The shuttle plunger 60 is actuated by a differential fluid pressure on its lower 62 and its upper 64 sides, in the dosing chamber 42, following a displacement of the slider 40 from an extreme position to the other.

The operation of the dispensing device according to the embodiment disclosed will be explained referring to figures 4 to 8.

Starting from the rest position illustrated on figure 4, where the slider 40 is in its upper position, the shuttle plunger 60 is in its lower position, and the dosing chamber 42 is in communication with the reservoir 14 through the upper inlet channel 46, a manual pressure is applied on the upper extremity of the slider 40 such that it slides down.

During its transversal displacement, the slider 40 comes to an intermediate non stable position, illustrated by figure 5, in which all its channels 46, 48, 50, 52 are closed by the sealing surfaces 70.

In this intermediate position, the shuttle plunger 60 stays down since the dosing chamber 42 is filled with fluid F.

At the end of this displacement, the slider 40 reaches its lower position, illustrated by figure 6, in which the lower inlet 48 matches with the lower channel 36 of the support member 28 establishing communication with the reservoir 14, and in which the upper outlet 50 emerges into the opening 54 of the front cap 32.

The lower side 62 of the shuttle plunger 60 is under fluid pressure of the reservoir 14 which causes the shuttle plunger 60 to slide up expelling the fluid F contained in the upper compartment of the dosing chamber 42 through the upper outlet 50 in the form of a stream S directed towards the eye 58 of the patient, while simultaneously inducing refilling of the lower compartment of the dosing chamber 42, as illustrated by figure 7.

Advantageously, the upper outlet 50 emerges into the opening 54 slightly before the opening of the lower inlet 48 such that, when the stream S of fluid F starts, the upper outlet 50 is completely open, in order to obtain an accurate and continuous stream S.

During stream S production, the piston 18 slides towards the dispersing front end 24 of the cartridge.

At the end of its displacement, the shuttle plunger 60 is in its upper position, as shown on figure 8, closing the upper outlet 50 and the upper inlet 46, and a new dose D of fluid F is ready in the lower compartment of the dosing chamber 42.

The quantity of fluid F expelled through the nozzle channel 50 corresponds to a dose D of fluid F.

Symmetrically, the next fluid injection can be realized pressing up the slider 40, as illustrated by the arrow on figure 8.

A correct alignment of the dispensing device 10 with the eye 58 is ensured when the patient can see the outlet 50, 52 through the opening 54. An additional visual alignment feature can be easily included along the dispensing device 10, for example a hole and a visible element in black or in another color, or a groove.

The delivery of the fluid F occurs in a single stream S within a time of approximately forty milliseconds for an "usual dose" in the range of thirty micro-liters. This timing is crucial to overcome the blink reflex.

Of course, dose range and time range for delivery could be different. Dose can be chosen by geometry, typically between five and fifty micro-liters. Time range is given by pressure, friction, and nozzle geometry and can be chosen typically between five and fifty milliseconds.

Thanks to adequate design of the spring 16, of the channels 34, 36, of the inlets 46, 48, of the outlets 50, 52, of the piston 18, and of the shuttle plunger 60, the delivery time varies only within an acceptable range when the reservoir 14 is being emptied.

The actuation speed of the slider 40 has no impact on the delivery time, neither on dose D. The slider 40 is provided with appropriate hard points to make stop impossible during opening of the inlets 46, 48 and of the outlets 50, 52.

The embodiment has been described with dispensing of fluid in the form of a stream S. The fluid F could also be dispensed in the form of a spray. The choice of a spray or a stream S depends on the medication to be delivered and can be chosen by adapting the nozzle shape.

Advantageously, the dispensing device 10 can be provided with a casing 72, improving ergonomics.

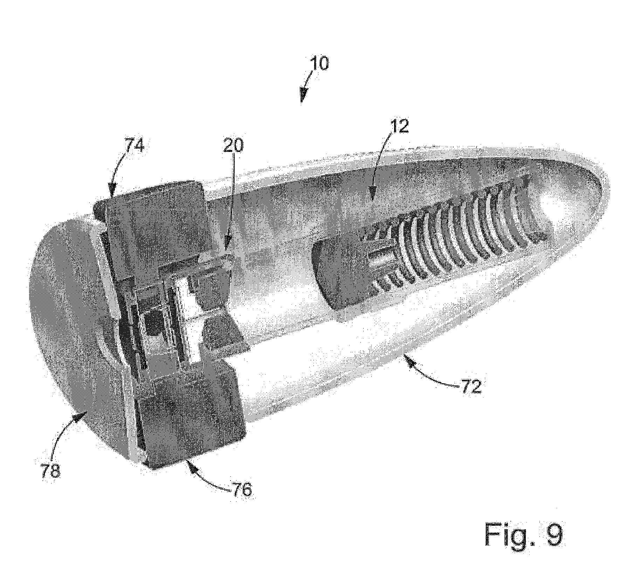

In the example shown on figure 9, the casing 72 is enveloping entirely the dosing module 20 and the cartridge 12. An upper 74 and a lower 76 press buttons are arranged on the dosing module 20 in order to permit actuation of the slider 40. A front disc 78, with a hole facing the opening 54 of the dosing module 20, closes the front end of the casing 72.

The casing 72 could also be provided with additional features (not shown) such as dose counter, timer, reminder, delivery tracking, etc.

It should be noted that, in a different -more sophisticated- embodiment, manual actuation can be replaced by an actuator of any type such as linear, electromagnetic, lever mechanism or whatever, which could be located in the casing 72.

## Claims

1. Dispensing device for applying fluid comprising a pressurized reservoir equipped with a dosing module, the dosing module having at least one dosing chamber which is able to communicate, on one hand, with the reservoir and, on the other hand, with at least one nozzle provided for dispensing a dose of fluid, **characterized in that** the dosing chamber comprises a moving and/or deformable member which delimits at least a first and a second compartments of variable volume, each compartment having an inlet and an outlet associated with valve means, wherein valve means are controlled according to a first configuration in which the inlet of the first compartment and the outlet of the second compartment are open while the other inlet and outlet being closed, and according to a second configuration in which the inlet of the second compartment and the outlet of the first compartment are open while the other inlet and outlet being closed, such that, in the first configuration, the first compartment is refilled with a dose of fluid, under reservoir pressure, while the dose of fluid contained in the second compartment is expelled and, in the second configuration, the second compartment is refilled with a dose of fluid, under reservoir pressure, while the dose of fluid contained in the first compartment is expelled.

2. Dispensing device according to the preceding claim, **characterized in that** the moving member is a shuttle plunger sliding in the dosing chamber between two extreme positions.

3. Dispensing device according to claim 1 or 2, **characterized in that** the valve means comprise a slide valve assembly which is able to open the inlet of the first compartment and the outlet of the second compartment, while the other inlet and outlet remain closed, and to open the inlet of the second compartment and the outlet of the first compartment, while the other inlet and outlet remain closed.

4. Dispensing device according to the preceding claim, **characterized in that** the slide valve assembly comprises a tubular slider delimiting internally the dosing chamber and which is slidably mounted on a support member of the dosing module between an upper and a lower positions, the slider comprising an upper and a lower inlet which are able to communicate with the reservoir, and an upper and a lower outlet which are able to communicate with an opening of the dosing module such that, when the slider is in its lower position, the upper inlet communicates with the dosing chamber and the lower outlet communicates with the opening, the other inlet and outlet being closed by sealing surfaces, and a lower position in which the lower inlet communicates with the dosing chamber and the upper outlet communicates with the opening, the other inlet and outlet being closed by sealing surfaces.

5. Dispensing device according to the preceding claim, **characterized in that**, the slider comprises a shuttle plunger which is slidably mounted in the dosing chamber between an upper position in which the shuttle plunger closes the upper outlet, and a lower position in which the shuttle plunger closes the lower outlet.

6. Dispensing device according to claim 4 or 5, **characterized in that** the reservoir comprises a piston biased towards the dispensing extremity of the reservoir.

7. Dispensing device according to the preceding claim, **characterized in that** the slider and the shuttle plunger are slidably mounted in a coaxial direction, transversally relatively to the sliding direction of the piston.

8. Dispensing device according to claim 6 or 7, **characterized in that** the piston is biased towards the dispensing extremity of the reservoir thanks to a pre-loaded spring.

9. Dispensing device according to anyone of the preceding claims, **characterized in that** the dispensing extremity of the reservoir is initially closed by a septum which is pierced during first use by a needle provided on the support member of the dosing module.

10. Dispensing device according to anyone of the preceding claims, **characterized in that** it comprises a casing in which the dosing module and the reservoir are housed.
